# EUROPÄISCHE PATENTSCHRIFT

(11) **EP 3 873 420 B1**
(45) Veröffentlichungstag und Bekanntmachung des Hinweises auf die Patenterteilung: **13.05.2026**
(21) Anmeldenummer: 19797674.9
(22) Anmeldetag: 31.10.2019
(51) Int. Cl.: A61K 8/58, A61Q 5/00, A61Q 5/02, A61Q 5/12

(54) **TENSID HALTIGES KOSMETISCHES MITTEL IN KOMBINATION MIT BIS(TRIETHOXYSILYLPROPYL)AMIN ZUR REINIGUNG UND PFLEGE VON HUMANHAAREN**
SURFACTANT-CONTAINING COSMETIC PRODUCT COMBINED WITH BIS(TRIETHOXYSILYLPROPYL) AMINE, FOR CLEANING AND CARE OF HUMAN HAIR
PRODUIT COSMÉTIQUE CONTENANT UN TENSIOACTIF, EN ASSOCIATION AVEC LA BIS(TRIÉTHOXYSILYLPROPYL)AMINE POUR LE LAVAGE ET LE SOIN DES CHEVEUX HUMAINS

(30) Priorität: 31.10.2018 DE 102018127273
(43) Veröffentlichungstag der Anmeldung: 08.09.2021
(73) Patentinhaber: Henkel AG & Co. KGaA, 40589 Düsseldorf (DE)
(72) Erfinder: KROHN, Rene, 22848 Norderstedt (DE); SCHULZE ZUR WIESCHE, Erik, 33619 Bielefeld (DE)
(86) Internationale Anmeldenummer: PCT/EP2019/079773
(87) Internationale Veröffentlichungsnummer: WO 2020/089359

(56) Entgegenhaltungen:
- EP-A2- 0 159 628
- EP-A2- 2 343 039
- WO-A1-2018/108919
- WO-A1-2019/002149
- DE-A1- 102014 205 806
- FR-A1- 2 954 100
- FR-A1- 2 966 351
- FR-A1- 2 976 800
- FR-A1- 3 044 904
- FR-A1- 3 060 326

## Beschreibung

Die vorliegende Erfindung betrifft kosmetische Mittel zur Behandlung eines keratinischen Materials, wobei das Mittel zwei organische Siliciumverbindungen und ein anionisches, nichtionisches oder amphoteres Tensid umfasst, sowie die Verwendung des kosmetischen Mittels.

Die äußere Beanspruchung der Haare durch chemische Stoffe aus einer Vielzahl unterschiedlicher Quellen stellt die Entwicklung kosmetischer Pflegeprodukte vor Herausforderungen. Luft- und Wasserverunreinigungen wirken sich nachteilig auf Haut und Haare aus. Zu den wichtigsten Luftschadstoffen gehören polycyclische aromatische Kohlenwasserstoffe, flüchtige organische Verbindungen, Stickoxide (NOx), Partikel und Zigarettenrauch. Die Wirkung verschiedener Luftschadstoffe kann in Gegenwart anderer Luftschadstoffe und unter Einwirkung von UV-Strahlung verstärkt werden.

Es ist bekannt, dass die Toxizität von gasförmigen Schadstoffen der Luft, wie Schwefeldioxid, Ozon und Stickoxiden, insbesondere mit ihrer Initiatoraktivität für freie Radikale zusammenhängt, die bei Lebewesen Schäden verursachen. Freie Radikale sind Stoffwechselprodukte, die auch natürlicherweise im Körper vorkommen. In grosser Menge können freie Radikale Irritationen und Entzündungen begünstigen und den Prozess der Alterung beschleunigen. In dem Fall spricht man von "oxidativem Schaden". Freie Radikale können auch eine Haarschädigung bewirken, die beispielsweise als Verringerung des Glanzes sowie des Griffs und/oder des Verblassens der Haarfarbe sichtbar wird.

Partikel sind eine komplexe Mischung, die Metalle, Mineralien, organische Toxine und/oder biologische Materialien enthalten. Auch sie können die Bildung von freien Radikalen begünstigen.

Ferner sind oft wechselnde Konsumentenwünsche hinsichtlich einer bestimmten Beschaffenheit der Haare mit einer wiederkehrenden chemischen Beanspruchung der Haare verbunden. Beispielsweise beanspruchen Haarfärbungen die Haare, aufgrund dessen eine besondere, intensive Pflege nötig sein kann.

Im Stand der Technik werden siliciumorganische Verbindungen aus der Gruppe der Silane beschrieben, die mindestens eine Hydroxygruppe und/oder hydrolysierbare Gruppe umfassen. Aufgrund der Anwesenheit der Hydroxygruppen und/oder hydrolysierbaren Gruppen handelt es sich bei den Silanen um reaktive Substanzen, die in Gegenwart von Wasser hydrolysieren bzw. oligomerisieren oder polymerisieren. Die durch Anwesenheit des Wassers initiierte Oligomerisierung oder Polymerisierung der Silane führt bei Anwendung auf einem keratinischen Material letztendlich zur Ausbildung eines Films, der eine Schutzwirkung entfalten kann.

Aus der FR 3060326 A1 ist ein kosmetisches Haarbehandlungsmittel bekannt, welches zwei organische Siliciumverbindungen, zwei verschiedene anionische Tenside, ein nichtionisches Tensid und ein amphoteres Tensid enthält.

Aus der FR 3 044 904 A1 ist ein kosmetisches Haarbehandlungsmittel bekannt, welches zwei organische Siliciumverbindungen, zwei verschiedene anionische Tenside, ein nichtionisches Tensid und ein amphoteres Tensid enthält.

Es besteht Bedarf an einem Produkt, das die schädlichen Auswirkungen von Luft- und Wasserverunreinigungen auf ein keratinisches Material, insbesondere Haare, oder die beanspruchenden Auswirkungen durch Haarbehandlungen auf ein keratinisches Material, insbesondere Haare, reduzieren oder verhindern kann.

Die der vorliegenden Erfindung zugrunde liegende Aufgabe besteht in der Bereitstellung eines Produkts mit einer verbesserten Pflege- und/oder Schutzwirkung.

Diese Aufgabe wird gelöst durch ein kosmetisches Mittel zur Behandlung eines keratinischen Materials, umfassend
a) mindestens eine organische Siliciumverbindung und
b) ein anionisches, nichtionisches und/oder amphoteres Tensid,
wobei das Mittel zur Behandlung eines keratinischen Materials- bezogen auf das Gesamtgewicht des Mittels zur Behandlung eines keratinischen Materials - enthält:
- 0,5 bis 3 Gew.-% mindestens einer ersten organischen Siliciumverbindung, die ausgewählt ist aus der Gruppe aus (3-Aminopropyl)trimethoxysilan, (3-Aminopropyl)triethoxysilan, (2-Aminoethyl)tri-methoxysilan, (2-Aminoethyl)triethoxysilan, (3-Dimethylaminopropyl)trimethoxysilan, (3-Dimethylaminopropyl)triethoxysilan (2-Dimethylaminoethyl)trimethoxysilan und (2-Dimethylaminoethyl)triethoxysilan, und
- 3,2 bis 7 Gew.-% mindestens einer zweiten organischen Siliciumverbindung, die ausgewählt ist aus der Gruppe aus Methyltrimethoxysilan, Methyltriethoxysilan, Ethyltrimethoxysilan, Ethyltriethoxysilan, Propyltrimethoxysilan, Propyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan, Octadecyltrimethoxysilan und Octadecyltriethoxysilan.

Unter einem keratinischen Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.

Bevorzugt werden unter einem keratinischen Material das menschliche Haar, die menschliche Haut und menschliche Nägel, insbesondere Finger- und Fußnägel, verstanden. Ganz besonders bevorzugt wird unter keratinischem Material das menschliche Haar, insbesondere Kopf- und/oder Barthaare, verstanden.

Als ersten erfindungswesentlichen Bestandteil enthält das kosmetische Mittel zur Behandlung eines keratinischen Materials mindestens zwei organische Siliciumverbindungen.

Organische Siliciumverbindungen, die alternativ auch als siliciumorganische Verbindungen bezeichnet werden, sind Verbindungen, die entweder eine direkte Silicium-Kohlenstoff-Bindung (Si-C) aufweisen oder in denen der Kohlenstoff über ein Sauerstoff-, Stickstoff- oder Schwefel-Atom an das Silicium-Atom geknüpft ist.

Die Bezeichnung Silan steht nach den IUPAC-Regeln für eine Stoffgruppe chemischer Verbindungen, die auf einem Silicium-Grundgerüst und Wasserstoff basieren. Bei organischen Silanen sind die WasserstoffAtome ganz oder teilweise durch organische Gruppen wie beispielsweise (substituierte) Alkylgruppen und/oder Alkoxygruppen ersetzt. In den organischen Silanen kann auch ein Teil der Wasserstoffatome durch Hydroxygruppen ersetzt sein.

Ganz besonders gute Ergebnisse konnten erhalten werden, wenn das Mittel zur Behandlung eines keratinischen Materials - bezogen auf das Gesamtgewicht des Mittels zur Behandlung eines keratinischen Materials - enthält:
- 0,5 bis 3 Gew.-% mindestens einer ersten organischen Siliciumverbindung, die ausgewählt ist aus der Gruppe aus (3-Aminopropyl)trimethoxysilan, (3-Aminopropyl)triethoxysilan, (2-Aminoethyl)tri-methoxysilan, (2-Aminoethyl)triethoxysilan, (3-Dimethylaminopropyl)trimethoxysilan, (3-Dimethylaminopropyl)triethoxysilan (2-Dimethylaminoethyl)trimethoxysilan und (2-Dimethylaminoethyl)triethoxysilan, und
- 3,2 bis 7 Gew.-% mindestens einer zweiten organischen Siliciumverbindung, die ausgewählt ist aus der Gruppe aus Methyltrimethoxysilan, Methyltriethoxysilan, Ethyltrimethoxysilan, Ethyltriethoxysilan, Propyltrimethoxysilan, Propyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan, Octadecyltrimethoxysilan und Octadecyltriethoxysilan . (3-Aminopropyl)trimethoxysilan kann beispielsweise von Sigma-Aldrich käuflich erworben werden. Auch (3-Aminopropyl)triethoxysilan ist kommerziell bei der Firma Sigma-Aldrich erhältlich.

Im Rahmen einer weiteren Ausführungsform enthält das Mittel zur Behandlung eines keratinischen Materials ferner mindestens eine organische Siliciumverbindung der Formel (II)

(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II).

Die siliciumorganischen Verbindungen der Formel (II) tragen jeweils an ihren beiden Enden die Siliciumhaltigen Gruppierungen (R₅O)_{c}(R₆)_{d}Si- und -Si(R₆')_{d'}(OR₅')_{c'}.

Im Mittelteil des Moleküls der Formel (II) befinden sich die Gruppierungen -(A)ₑ- und -[NR₇-(A')]_{f}- und -[O-(A")]_{g}- und -[NR₈-(A‴)]ₕ-. Hierbei kann jeder der Reste e, f, g und h unabhängig voneinander für die Zahl 0 oder 1 stehen, wobei die Maßgabe besteht, dass mindestens einer der Reste e, f, g und h von 0 verschieden ist. Mit anderen Worten enthält eine organischen Siliciumverbindung der Formel (II) mindestens eine Gruppierung aus der Gruppe aus -(A)- und -[NR₇-(A')]- und -[O-(A")]- und -[NR₈-(A‴)]-.

In den beiden endständigen Struktureinheiten (R₅O)_{c}(R₆)_{d}Si- und - Si(R₆')_{d'}(OR₅')_{c'} stehen die Reste R5, R5', R5" unabhängig voneinander für ein Wasserstoffatom oder für eine C₁-C₆-Alkylgruppe. Die Reste R6, R6' und R6" stehen unabhängig voneinander für eine C₁-C₆-Alkylgruppe.

Hierbei steht c für eine ganze Zahl von 1 bis 3, und d steht für die ganze Zahl 3 - c. Wenn c für die Zahl 3 steht, dann ist d gleich 0. Wenn c für die Zahl 2 steht, dann ist d gleich 1. Wenn c für die Zahl 1 steht, dann ist d gleich 2.

Analog steht c' für eine ganze Zahl von 1 bis 3, und d' steht für die ganze Zahl 3 - c'. Wenn c' für die Zahl 3 steht, dann ist d' gleich 0. Wenn c' für die Zahl 2 steht, dann ist d' gleich 1. Wenn c' für die Zahl 1 steht, dann ist d' gleich 2.

Eine sehr hohe Anti-Pollution-Wirkung des Mittels zur Behandlung eines keratinischen Materials konnte erhalten werden, wenn die Reste c und c' beide für die Zahl 3 stehen. In diesem Fall stehen d und d' beide für die Zahl 0.

In einer weiteren bevorzugten enthält das Mittel zur Behandlung eines keratinischen Materials mindestens eine organische Siliciumverbindung der Formel (II)

(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),

wobei
- R5 und R5' unabhängig voneinander für eine Methylgruppe oder eine Ethylgruppe stehen,
- c und c' beide für die Zahl 3 stehen und
- d und d' beide für die Zahl 0 stehen.

Wenn c und c' beide für die Zahl 3 stehen und d und d' beide für die Zahl 0 stehen, entsprechen die organischen Siliciumverbindung der Formel (IIa)

(R₅O)₃Si-(A)ₑ-[NR₇-(A')]_{f}-[O-(A")]_{g}-[NR₈-(A‴)]ₕ-Si(OR₅')₃ (IIa).

Die Reste e, f, g und h können unabhängig voneinander für die Zahl 0 oder 1 stehen, wobei mindestens ein Rest aus e, f, g und h von null verschieden ist. Durch die Kürzel e, f, g und h wird demnach definiert, welche der Gruppierungen -(A)ₑ- und -[NR₇-(A')]_{f}- und -[O-(A")]_{g}- und -[NR₈-(A‴)]ₕ-sich im Mittelteil der organischen Siliciumverbindung der Formel (II) befinden.

In diesem Zusammenhang hat sich die Anwesenheit bestimmter Gruppierungen als besonders vorteilhaft im Hinblick auf die Erhöhung der "Anti-Pollution"-Wirkung erwiesen. Besonders gute Ergebnisse konnten erhalten werden, wenn mindestens zwei der Reste e, f, g und h für die Zahl 1 stehen. Ganz besonders bevorzugt stehen e und f beide für die Zahl 1. Weiterhin ganz besonders bevorzugt stehen g und h beide für die Zahl 0.

Wenn e und f beide für die Zahl 1 stehen und g und h beide für die Zahl 0 stehen, entsprechen die organischen Siliciumverbindung der Formel (IIb)

(R₅O)_{c}(R₆)_{d}Si-(A)-[NR₇-(A')]-Si(R₆')_{d'}(OR₅')_{c'} (IIb).

Die Reste A, A', A", A‴ und Aʺʺ stehen unabhängig voneinander für eine lineare oder verzweigte, zweibindige C₁-C₂₀-Alkylengruppe. Bevorzugt stehen die Reste A, A', A", A‴ und Aʺʺ unabhängig voneinander für eine lineare, zweibindige C₁-C₂₀-Alkylengruppe. Weiter bevorzugt stehen die Reste A, A', A", A‴ und Aʺʺ unabhängig voneinander für eine lineare zweibindige C₁-C₆-Alkylengruppe. Besonders bevorzugt stehen die Reste A, A', A", A‴ und Aʺʺ unabhängig voneinander für eine Methylengruppe (-CH₂-), eine Ethylengruppe (-CH₂-CH₂-), eine Propylengruppe (-CH₂-CH₂-CH₂-) oder eine Butylengruppe (-CH₂-CH₂-CH₂-CH₂-). Ganz besonders bevorzugt stehen die Reste A, A', A", A‴ und Aʺʺ für eine Propylengruppe (-CH₂-CH₂-CH₂-).

Wenn der Rest f für die Zahl 1 steht, dann enthält die organische Siliciumverbindung der Formel (II) eine strukturelle Gruppierung -[NR₇-(A')]-.

Wenn der Rest h für die Zahl 1 steht, dann enthält die organische Siliciumverbindung der Formel (II) eine strukturelle Gruppierung -[NR₈-(A‴)]-.

Hierbei stehen die Reste R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-Alkylgruppe oder eine Gruppierung der Formel (III)

- (Aʺʺ)-Si(R₆")_{d}"(OR₅")_{c}" (III).

Ganz besonders bevorzugt stehen die Reste R₇ und R₈ unabhängig voneinander für ein Wasserstoffatom, eine Methylgruppe, eine 2-Hydroxyethylgruppe, eine 2-Alkenylgruppe, eine 2-Aminoethylgruppe oder für eine Gruppierung der Formel (III).

Wenn der Rest f für die Zahl 1 steht und der Rest h für die Zahl 0 steht, enthält die organische Siliciumverbindung die Gruppierung [NR₇-(A')], aber nicht die Gruppierung -[NR₈-(A‴)]. Steht nun der Rest R7 für eine Gruppierung der Formel (III), so enthält das Mittel zur Behandlung eines keratinischen Materials eine organische Siliciumverbindung mit 3 reaktiven Silan-Gruppen.

In einer weiteren bevorzugten enthält das Mittel zur Behandlung eines keratinischen Materials mindestens eine organische Siliciumverbindung der Formel (II)

(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),

wobei
- e und f beide für die Zahl 1 stehen,
- g und h beide für die Zahl 0 stehen,
- A und A' unabhängig voneinander für eine lineare, zweibindige C₁-C₆-Alkylengruppe stehen
   und
- R7 für ein Wasserstoffatom, eine Methylgruppe, eine 2-Hydroxyethylgruppe, eine 2-Alkenylgruppe, eine 2-Aminoethylgruppe oder für eine Gruppierung der Formel (III) steht.

In einer weiteren bevorzugten Ausführungsform enthält das Mittel zur Behandlung eines keratinischen Materials mindestens eine organische Siliciumverbindung der Formel (II), wobei
- e und f beide für die Zahl 1 stehen,
- g und h beide für die Zahl 0 stehen,
- A und A' unabhängig voneinander für eine Methylengruppe (-CH₂-), eine Ethylengruppe (-CH₂-CH₂-) oder eine Propylengruppe (-CH₂-CH₂-CH₂) stehen,
   und
- R₇ für ein Wasserstoffatom, eine Methylgruppe, eine 2-Hydroxyethylgruppe, eine 2-Alkenylgruppe, eine 2-Aminoethylgruppe oder für eine Gruppierung der Formel (III) steht.

Zur Lösung der Aufgabenstellung gut geeignete organische Siliciumverbindungen der Formel (II) sind
- 3-(Trimethoxysilyl)-N-[3-(trimethoxysilyl)propyl]-1-propanamin 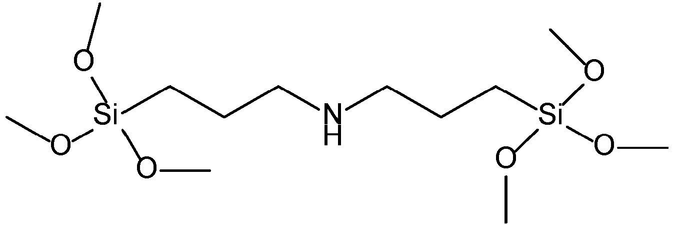
- 3-(Triethoxysilyl)-N-[3-(triethoxysilyl)propyl]-1-propanamin 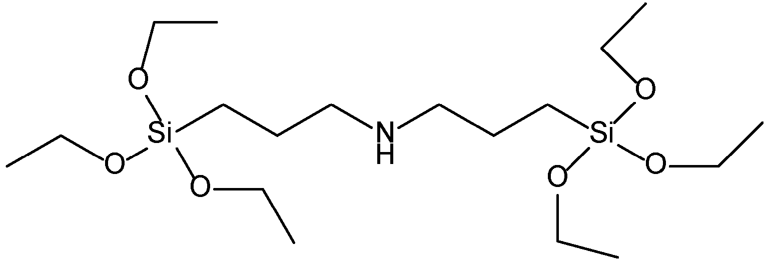
-N-Methyl-3-(trimethoxysilyl)-N-[3-(trimethoxysilyl)propyl]-1-propanamin 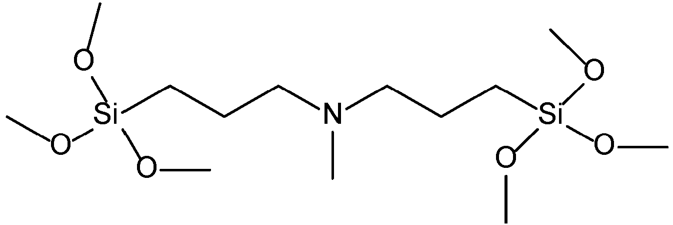
-N-Methyl-3-(triethoxysilyl)-N-[3-(triethoxysilyl)propyl]-1-propanamin 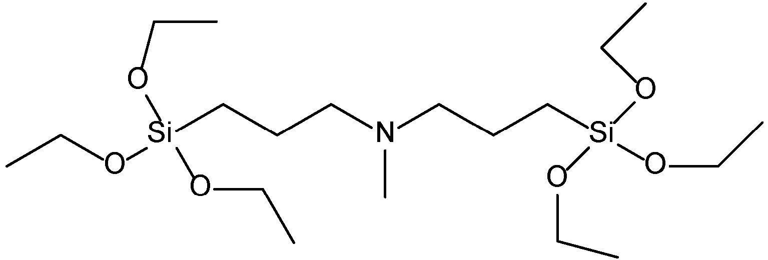
- 2-[Bis[3-(trimethoxysilyl)propyl]amino]-ethanol 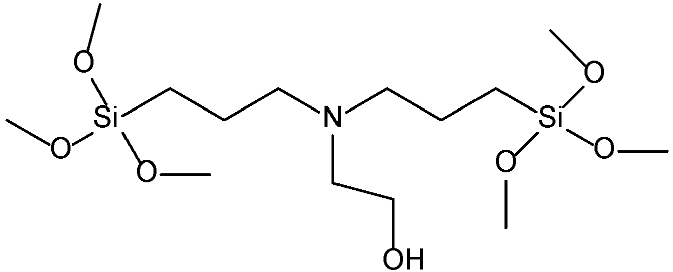
- 2-[Bis[3-(triethoxysilyl)propyl]amino]-ethanol 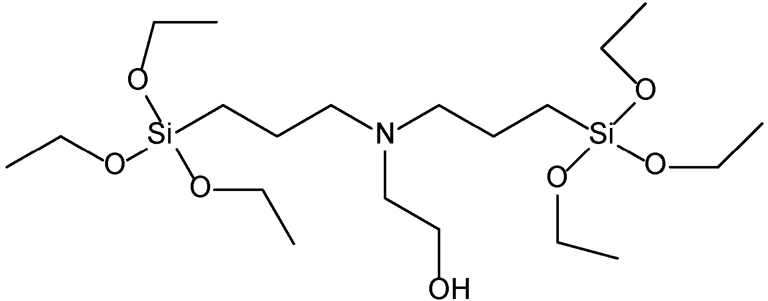
- 3-(Trimethoxysilyl)-N,N-bis[3-(trimethoxysilyl)propyl]-1-propanamin 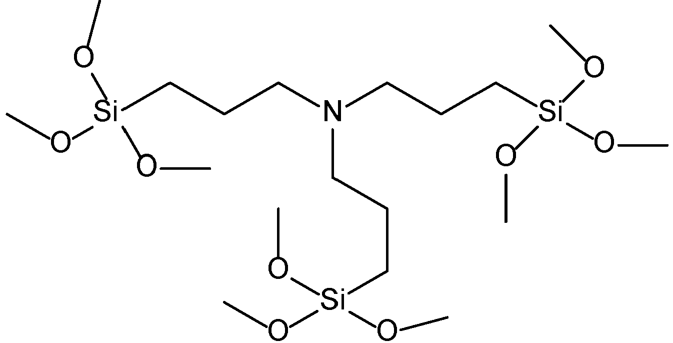
- 3-(Triethoxysilyl)-N,N-bis[3-(triethoxysilyl)propyl]-1-propanamin 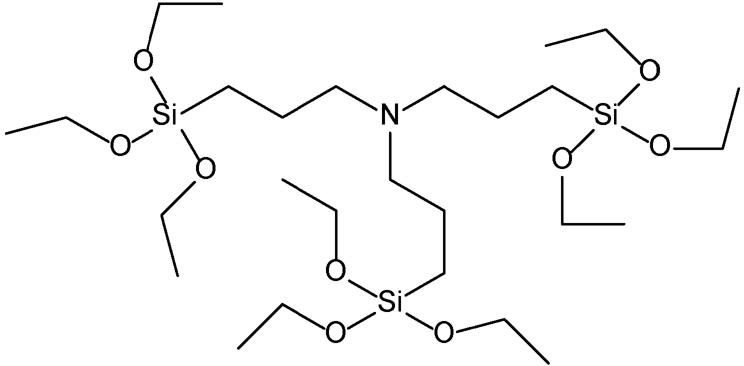
- N1,N1-Bis[3-(trimethoxysilyl)propyl]-1,2-ethanediamin, 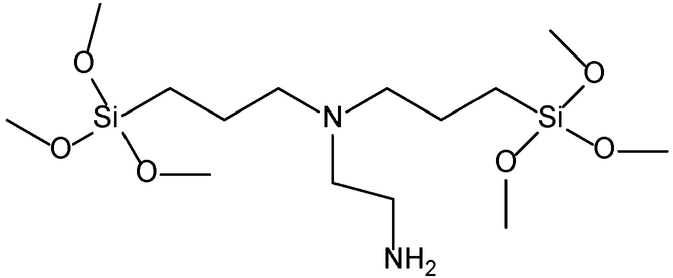
- N1,N1-Bis[3-(triethoxysilyl)propyl]-1,2-ethanediamin, 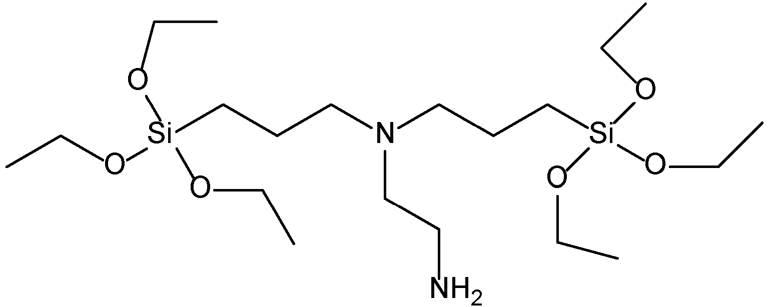
- N,N-Bis[3-(trimethoxysilyl)propyl]-2-propen-1-amin 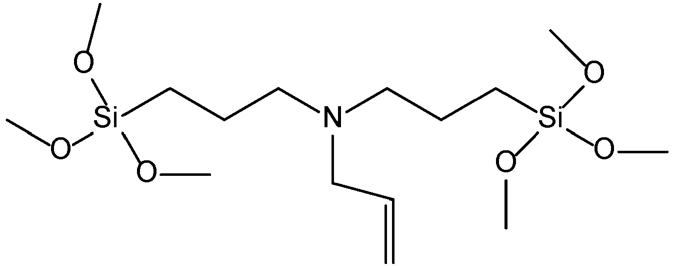
- N,N-Bis[3-(triethoxysilyl)propyl]-2-propen-1-amin

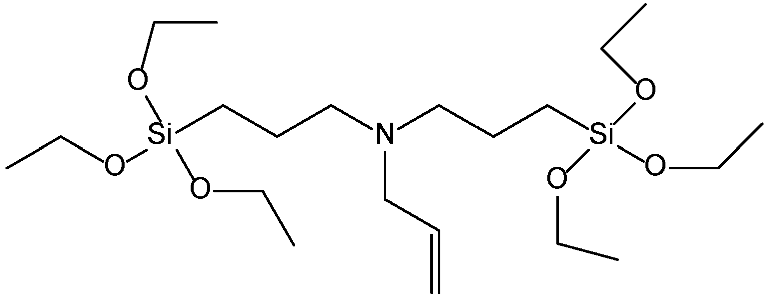

Die vorgenannten organische Siliciumverbindung der Formel (II) sind kommerziell erhältlich.

Bis(trimethoxysilylpropyl)amin mit der CAS-Nummer 82985-35-1 kann beispielsweise von Sigma-Aldrich käuflich erworben werden.

Bis[3-(triethoxysilyl)propyl]amin, auch bezeichnet als 3-(Triethoxysilyl)-N-[3-(triethoxysilyl)propyl]-1-propanamin, mit der CAS-Nummer 13497-18-2 kann zum Beispiel von Sigma-Aldrich käuflich erworben werden oder ist im Handel unter der Produktbezeichnung Dynasylan 1122 von Evonik erhältlich.

N-Methyl-3-(trimethoxysilyl)-N-[3-(trimethoxysilyl)propyl]- 1-propanamin wird alternativ auch als Bis(3-trimethoxysilylpropyl)-N-methylamin bezeichnet und kann bei Sigma-Aldrich oder Fluorochem kommerziell erworben werden.

3-(Triethoxysilyl)-N,N-bis[3-(triethoxysilyl)propyl]-1-propanamin mit der CAS-Nummer 18784-74-2 kann beispielsweise von Fluorochem oder Sigma-Aldrich käuflich erworben werden.

Bei den zuvor beschriebenen organischen Siliciumverbindungen handelt es sich um reaktive Verbindungen.

In einer explizit ganz besonders bevorzugten Ausführungsform ist ein Mittel dadurch gekennzeichnet, dass es mindestens eine organische Silicumverbindunge enthält, welche aus der Gruppe aus (3-Aminopropyl)triethoxysilan und (3-Aminopropyl)trimethoxysilan ausgewählt ist, und mindestens eine organische Silicumverbindungen enthält, welche aus der Gruppe bestehend aus Methyltrimethoxysilan, Methyltriethoxysilan, Ethyltrimethoxysilan, Ethyltriethoxysilan, Propyltrimethoxysilan, Propyltriethoxysilan, Hexyltrimethoxysilan und Hexyltriethoxysilan ausgewählt ist.

Bereits der Zusatz geringer Wassermengen führt bei organischen Siliciumverbindungen mit mindestens einer hydrolysierbaren Gruppe zur Hydrolyse. Die Hydrolyseprodukte und/oder organischen Siliciumverbindungen mit mindestens einer Hydroxygruppe können in einer Kondensationsreaktion miteinander reagieren. Aus diesem Grund können sowohl die siliciumorganischen Verbindungen mit mindestens einer hydrolysierbaren Gruppe als auch deren Hydrolyse- und/oder Kondensationsprodukte in dem Mittel enthalten sein. Bei Verwendung von siliciumorganischen Verbindungen mit mindestens einer Hydroxylgruppe können sowohl die organischen Siliciumverbindungen mit mindestens einer Hydroxylgruppe als auch deren Kondensationsprodukte in dem Mittel enthalten sein.

Unter einem Kondensationsprodukt wird ein Produkt verstanden, dass durch Reaktion von mindestens zwei organischen Siliciumverbindungen mit jeweils mindestens einer Hydroxylgruppen oder hydrolysierbaren Gruppen pro Molekül unter Abspaltung von Wasser und/oder unter Abspaltung von einem Alkanol entsteht. Die Kondensationsprodukte können beispielsweise Dimere, aber auch Trimere oder Oligomere sein, wobei die Kondensationsprodukte mit den Monomeren im Gleichgewicht stehen. Abhängig von der eingesetzten oder in der Hydrolyse verbrauchten Wassermenge verschiebt sich das Gleichgewicht von monomerer organischen Siliciumverbindungen zu Kondensationsprodukt.

Im Rahmen der vorliegenden Erfindung sind Angaben in Gew.-% - falls nicht anders angegeben - immer bezogen auf das Gesamtgewicht des kosmetischen Mittels.

Als zweiten erfindungswesentlichen Bestandteil enthält das kosmetische Mittel zur Behandlung eines keratinischen Materials ein anionisches, nichtionisches oder amphoteres Tensid. Im Zuge der zu dieser Erfindung führenden Arbeiten hat sich herausgestellt, dass zur Erzielung einer besonders guten Pflege-Wirkung es insbesondere von Vorteil ist, wenn die organische Siliciumverbindung, beispielsweise (3-Aminopropyl)trimethoxysilan oder (3-Aminopropyl)triethoxysilan, mit einem anionischen, nichtionischen und/oder amphoteren Tensid kombiniert wird.

Es wurde herausgefunden, dass die Kombination aus den Tensiden und der organischen Siliciumverbindung besonders leistungsstark in Bezug auf die Pflege ist. Die Haaroberfläche wird hydrophobisiert und damit Frizz reduziert. Das Haar wird weicher und selbst bei einer hohen chemischen Beanspruchung lässt sich das Haar trotzdem besser formen.

Gemäß einer bevorzugten Ausführungsform der vorliegenden Erfindung sind ein oder mehrere Tenside in dem kosmetischen Mittel enthalten, die zusammen eine Gesamtmenge an Tensid bilden. Gemäß dieser bevorzugten Ausführungsform beträgt die Gesamtmenge an Tensid 1 bis 30 Gew.-%, bevorzugt 2 bis 25 Gew.-%, bevorzugter 3 bis 20 Gew.-%, bezogen auf das Gesamtgewicht des kosmetischen Mittels.

Gemäß einer bevorzugten Ausführungsform der vorliegenden Erfindung sind die anionischen Tenside in dem kosmetischen Mittel ausgewählt ist aus der Gruppe bestehend aus
- geradkettigen oder verzweigten, gesättigten oder ein- oder mehrfach ungesättigten Alkylsulfonaten mit 8 bis 24, bevorzugt 12 bis 22, bevorzugter 16 bis 18 C-Atomen,
- linearen Alpha-Olefinsulfonaten mit 8 bis 24, bevorzugt 12 bis 22, bevorzugter 16 bis 18 C-Atomen,
- Alkylsulfaten und Alkylpolyglykolethersulfaten der Formel R₉-O-(CH₂-CH₂O)ₙ-SO₃X, in der R₉ bevorzugt für einen geradkettigen oder verzweigten, gesättigten oder ein- oder mehrfach ungesättigten Alkyl- oder Alkenlyrest mit 8 bis 24, bevorzugt 12 bis 22, bevorzugter 16 bis 18 Kohlenstoffatomen, n für 0 oder 1 bis 12, bevorzugter 2 bis 4 und X für ein Alkali- oder Erdalkalimetallion oder für protoniertes Triethanolamin oder das Ammonium-Ion steht,
- geradkettigen oder verzweigten, gesättigten oder ein- oder mehrfach ungesättigten Alkylcarbonsäuren mit 8 bis 24, bevorzugt 12 bis 22, bevorzugter 16 bis 18 C-Atomen,
- geradkettigen oder verzweigten, gesättigten oder ein- oder mehrfach ungesättigten Alkylphosphaten mit 8 bis 24, bevorzugt 12 bis 22, bevorzugter 16 bis 18 C-Atomen,
- Alkylisethionat, dessen Alkylgruppe ausgewählt ist aus einer verzweigten oder unverzweigten C₆ bis C₂₂, bevorzugt C₁₀ bis C₁₈, bevorzugter C₁₂ bis C₁₆ Alkylgruppe, insbesondere Natriumcocoylisethionat,
- Alkylglycosidcarbonsäuren, dessen Alkylgruppe ausgewählt ist aus einer verzweigten oder unverzweigten C₆ bis C₂₂, bevorzugt C₁₀ bis C₁₈, bevorzugter C₁₂ bis C₁₆ Alkylgruppe,
- Alkylsulfosuccinaten, dessen zwei Alkylgruppen ausgewählt sind aus gleichen oder verschiedenen, verzweigte oder unverzweigten C₂ bis C₁₂, bevorzugt C₄ bis C₁₀, bevorzugter C₆ bis C₈ Alkylgruppen,
- Alkyltauraten, dessen Alkylgruppe ausgewählt ist aus einer verzweigten oder unverzweigten C₆ bis C₂₂, bevorzugt C₁₀ bis C₁₈, bevorzugter C₁₂ bis C₁₆ Alkylgruppe,
- Alkylsarcosinaten, dessen Alkylgruppe ausgewählt ist aus einer verzweigten oder unverzweigten C₆ bis C₂₂, bevorzugt C₁₀ bis C₁₈, bevorzugter C₁₂ bis C₁₆ Alkylgruppe,
- Sulfonate ungesättigter Fettsäuren mit 8 bis 24, bevorzugt 12 bis 22, bevorzugter 16 bis 18 C-Atomen und 1 bis 6 Doppelbindungen,
wobei das Gegenion des anionischen Tensids ein Alkali- oder Erdalkalimetallion oder ein protoniertes Triethanolamin oder das Ammonium-Ion ist.

Besonders bevorzugte anionische Tenside sind geradkettige oder verzweigte Alkylethersulfate, die einen Alkylrest mit 8 bis 18 und insbesondere mit 10 bis 16 C-Atomen sowie 1 bis 6 und insbesondere 2 bis 4 Ethylenoxideinheiten enthalten. Ganz besonders bevorzugt enthält die Tensidmischung aus anionischen und amphoteren/zwitterionischen Tensiden Natriumlaurylethersulfat (INCI: Sodium Laureth Sulfate) und ganz besonders bevorzugt Natriumlaurylethersulfat mit 2 Ethylenoxideinheiten.

Amphotere Tenside, welche auch als zwitterionische Tenside bezeichnet werden, werden solche oberflächenaktiven Verbindungen genannt, die im Molekül mindestens eine quartäre Ammoniumgruppe und mindestens eine -COO⁻ - oder -SO₃⁻ -Gruppe tragen. Unter amphoteren/zwitterionischen Tensiden werden auch solche oberflächenaktiven Verbindungen verstanden, die außer einer C₈ - C₂₄ -Alkyl- oder - Acylgruppe mindestens eine freie Aminogruppe und mindestens eine -COOH- oder -SO₃H-Gruppe enthalten und zur Ausbildung innerer Salze befähigt sind.

Gemäß einer bevorzugten Ausführungsform der vorliegenden Erfindung sind die amphoteren Tenside in dem kosmetischen Mittel ausgewählt aus der Gruppe bestehend aus
- Alkylbetain, umfassend mindestens eine gesättigte oder ungesättigte, verzweigte oder unverzweigte C₆ bis C₂₂, bevorzugt C₁₀ bis C₁₈, bevorzugter C₁₂ bis C₁₆ Alkylgruppe,
- Alkylamphodiacetat oder Alkylamphodiacetat, umfassend eine gesättigte oder ungesättigte, verzweigte oder unverzweigte C₆ bis C₂₂, bevorzugt C₁₀ bis C₁₈, bevorzugter C₁₂ bis C₁₆ Alkylgruppe, mit einem Alkali- oder Erdalkalimetallgegenion, und
- Alkylamidopropylbetain, umfassend mindestens eine gesättigte oder ungesättigte, verzweigte oder unverzweigte C₆ bis C₂₂, bevorzugt C₁₀ bis C₁₈, bevorzugter C₁₂ bis C₁₆ Alkylgruppe.

Zu den insbesondere geeigneten amphoteren/zwitterionischen Tensiden zählen die unter der INCI-Bezeichnung bekannten Tenside Cocamidopropylbetain und Disodium Cocoamphodiacetate.

Gemäß einer bevorzugten Ausführungsform der vorliegenden Erfindung ist das nichtionische Tensid ausgewählt aus der Gruppe bestehend aus
- Alkylglucamid, umfassend eine gesättigte oder ungesättigte, verzweigte oder unverzweigte C₆ bis C₂₂, bevorzugt C₁₀ bis C₁₈, bevorzugter C₁₂ bis C₁₆ Alkylgruppe,
- Alkylfructosid, umfassend eine gesättigte oder ungesättigte, verzweigte oder unverzweigte C₆ bis C₂₂, bevorzugt C₁₀ bis C₁₈, bevorzugter C₁₂ bis C₁₆ Alkylgruppe,
- Alkylglucosid, umfassend eine gesättigte oder ungesättigte, verzweigte oder unverzweigte C₆ bis C₂₂, bevorzugt C₁₀ bis C₁₈, bevorzugter C₁₂ bis C₁₆ Alkylgruppe,
- Alkylalkoholalkoxylat der Formel R₁₀(OR₁₁)ₘOH, in der R₁₀ eine lineare oder verzweigte C₆ bis C₂₂, bevorzugt C₁₀ bis C₁₈, bevorzugter C₁₂ bis C₁₆ Alkylgruppe, R₁₁ eine C₂ bis C₄, bevorzugt eine C₂ Alkylgruppe, und m 1 bis 10, bevorzugt 2 bis 6, bevorzugter 2 bis 6, darstellen, und
- Alkylester der Formel R₁₂COOR₁₃, in der R₁₂ eine lineare oder verzweigte C₆ bis C₂₂, bevorzugt C₁₀ bis C₁₈, bevorzugter C₁₂ bis C₁₆ Alkylgruppe, R₁₃ eine C₁ bis C₄, bevorzugt eine C₂ Alkylgruppe, darstellen.

Gemäß einer bevorzugten Ausführungsform der vorliegenden Erfindung enthält das kosmetische Mittel zwei strukturell voneinander verschiedene Tenside. Es ist insbesondere bevorzugt, dass das kosmetische Mittel zwei strukturell voneinander verschiedene anionische Tenside enthält, oder dass das kosmetische Mittel ein anionisches Tensid und ein nichtionisches Tensid enthält, oder dass das kosmetische Mittel ein anionisches Tensid und ein amphoteres Tensid enthält.

In einer explizit ganz besonders bevorzugten Ausführungsform enthält das Mittel zur Behandlung eines keratinischen Materials 3-(Triethoxysilyl)-N-[3-(triethoxysilyl)propyl]-1-propanamin und zwei strukturell voneinander verschiedene anionische Tenside oder ein anionisches Tensid in Kombination mit einem nichtionischen Tensid oder ein anionisches Tensid in Kombination mit einem amphoteren Tensid.

Das Mittel zur Behandlung eines keratinischen Materials kann insbesondere ein Mittel zur Reinigung eines keratinischen Materials, ein Mittel zur Pflege eines keratinischen Materials, ein Mittel zur Pflege und Reinigung eines keratinischen Materials und/oder ein Mittel zum temporären Umformen eines keratinischen Materials umfassen.

Im Folgenden werden weitere Bestandteile der Haarbehandlungsmittel beschrieben, die neben den zuvor beschriebenen zwingenden Inhaltstoffen in den Mitteln enthalten sein können.

Es kann bevorzugt sein, dass das Mittel zur Behandlung eines keratinischen Materials ferner 0,001 bis 20 Gew.-% mindestens einer quaternären Verbindung umfasst. Dies gilt insbesondere für Mittel zur Pflege eines keratinischen Materials und für Mittel zur Pflege und Reinigung eines keratinischen Materials.

Es ist bevorzugt, dass die mindestens eine quaternäre Verbindung ausgewählt ist aus mindestens einer der Gruppen bestehend aus
i) der Monoalkylquats und/oder
ii) der Esterquats und/oder
iii) der quaternären Imidazoline der Formel (Tkat2), 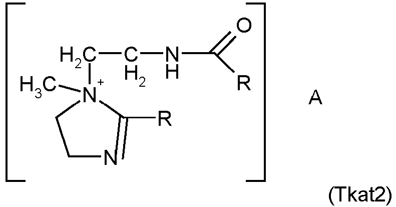 in welcher die Reste R unabhängig voneinander jeweils für einen gesättigten oder ungesättigten, linearen oder verzweigten Kohlenwasserstoffrest mit einer Kettenlänge von 8 bis 30 Kohlenstoffatomen und A für ein physiologisch verträgliches Anion steht, und/oder
iv) der Amidoamine und/oder kationisierten Amidoamine und/oder
v) Poly(methacryloyloxyethyltrimethylammoniumverbindungen) und/oder;
vi) quaternisierten Cellulose-Derivaten, insbesondere Polyquaternium 10, Polyquaternium-24, Polyquaternium-27, Polyquaternium-67, Polyquaternium-72, und/oder
vii) kationischen Alkylpolyglycosiden und/oder
viii) kationisiertem Honig und/oder
ix) kationischen Guar-Derivaten und/oder
x) Chitosan und/oder
xi) polymeren Dimethyldiallylammoniumsalzen und deren Copolymeren mit Estern und Amiden von Acrylsäure und Methacrylsäure, insbesondere Polyquaternium-7 und/oder
xii) Copolymeren des Vinylpyrrolidons mit quaternierten Derivaten des Dialkylaminoalkylacrylats und -methacrylats, insbesondere Polyquaternium-11 und/oder
xiii) Vinylpyrrolidon-Vinylimidazoliummethochlorid-Copolymeren, insbesondere Polyquaternium-16 und/oder
xiv) quaterniertem Polyvinylalkohol und/oder
xv) Polyquaternium-74,
sowie Mischungen hiervon.

Es ist insbesondere bevorzugt, dass das Haarbehandlungsmittel ein Monoalkylquat, ein Amidoamin und/oder ein kationisches Hompolymer, welches unter die INCI-Bezeichnung Polyquaternium-37 fällt, als quaternäre Verbindungen enthält.

Es kann bevorzugt sein, dass das Mittel zur Behandlung eines keratinischen Materials ferner eine festigende Verbindung, vorzugsweise ausgewählt aus der Gruppe bestehend aus Wachsen, synthetischen Polymeren und Mischungen daraus, umfasst.

Um den unterschiedlichen Anforderungen an Mittel zur Behandlung eines keratinischen Materials in Form eines Mittels zum temporären Umformen eines keratinischen Materials (= Stylingmittel) gerecht zu werden, sind als festigende Verbindungen bereits eine Vielzahl von synthetischen Polymeren entwickelt worden, die in dem Mittel zur Behandlung eines keratinischen Materials zur Anwendung kommen können. Alternativ oder ergänzend werden Wachse als festigende Verbindungen eingesetzt. Idealerweise ergeben die Polymere und/oder Wachse bei der Anwendung auf dem keratinischen Material einen Polymerfilm oder Film, der einerseits der Frisur einen starken Halt verleiht, andererseits aber hinreichend flexibel ist, um bei Beanspruchung nicht zu brechen.

Die synthetischen Polymere lassen sich in kationische, anionische, nichtionische und amphotere festigende Polymere unterteilen.

Geeignete synthetische Polymere umfassen beispielsweise Polymere mit den folgenden INCI-Bezeichnungen: Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Octylacrylamide/Diphenyl Amodimethicone Copolymer, Acrylates/Stearyl Acrylate/Ethylamine Oxide Methacrylate Copolymer, Acrylates/VA Copolymer, Acrylates/Hydroxyesters Acrylates Copolymer, AcrylatesNP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis- Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/ Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/Hydroxyethylmaleimide Copolymer, Isobutylene/MA Copolymer, Isobutylmethacrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, Octylacrylamide/Acrylates/Butylaminoethyl Methacrylate Copolymer, PEG/PPG-25/25 Dimethicone/Acrylates Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP, PVP/VA/Itaconic Acid Copolymer, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilylcarbamoyl Pullulan, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl Caprolactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VP/VA Copolymer, VP/Vinyl Caprolactam/DMAPA Acrylates Copolymer, Yeast Palmitate und Styrene/VP Copolymer. Ebenso geeinet sind Celluloseether, wie Hydroxypropylcellulose, Hydroxyethylcellulose und Methylhydroxypropylcellulose.

Auch Homopolyacrylsäure (INCI: Carbomer), die im Handel unter dem Namen Carbopol^{®} in unterschiedlichen Ausführungen erhältlich ist, ist als festigende Verbindung geeignet.

Bevorzugt umfasst die festigende Verbindung ein Vinylpyrrolidon-haltiges Polymer. Besonders bevorzugt umfasst die festigende Verbindung ein Polymer ausgewählt aus der Gruppe bestehend aus Polyvinylpyrrolidon (PVP), Vinylpyrrolidon-Vinylacetat-Copolymer (VP/VA Coplymer), Vinyl Caprolactam/VP/Dimethylaminoethyl Methacrylate Copolymer (INCI), VP/DMAPA Acrylates Copolymer (INCI) und Mischungen daraus.

Eine ebenfalls bevorzugte festigende Verbindung ist Octylacrylamide/Acrylates/Butylaminoethyl Methacrylate Copolymer (INCI), welches unter der Bezeichnung "Amphomer^{®}" von Akzo Nobel vertrieben wird.

Entsprechend ist es besonders bevorzugt, dass die festigende Verbindung ein synthetisches Polymer ausgewählt aus der Gruppe bestehend aus Polyvinylpyrrolidon (PVP), Vinylpyrrolidon-Vinylacetat-Copolymer (VP/VA Coplymer), Vinyl Caprolactam/VP/Dimethylaminoethyl Methacrylate Copolymer (INCI), VP/DMAPA Acrylates Copolymer (INCI), Octylacrylamide/Acrylates/Butylaminoethyl Methacrylate Copolymer (INCI) und Mischungen daraus umfasst.

Die kosmetische Zusammensetzung kann zusätzlich oder alternativ zu einem synthetischen Polymer mindestens ein natürliches oder synthetisches Wachs, welches einen Schmelzpunkt von über 37 °C aufweist, als festigende Verbindung enthalten.

Als natürliche oder synthetische Wachse können feste Paraffine oder Isoparaffine, Pflanzenwachse wie Candelillawachs, Carnaubawachs, Espartograswachs, Japanwachs, Korkwachs, Zuckerrohrwachs, Ouricurywachs, Montanwachs, Sonnenblumenwachs, Fruchtwachse und tierische Wachse, wie zum Beispiel Bienenwachse und andere Insektenwachse, Walrat, Schellackwachs, Wollwachs und Bürzelfett, weiterhin Mineralwachse, wie zum Beispiel Ceresin und Ozokerit oder die petrochemischen Wachse, wie zum Beispiel Petrolatum, Paraffinwachse, Microwachse aus Polyethylen oder Polypropylen und Polyethylenglycolwachse eingesetzt werden. Es kann vorteilhaft sein, hydrierte oder gehärtete Wachse einzusetzen. Weiterhin sind auch chemisch modifizierte Wachse, insbesondere die Hartwachse, zum Beispiel Montanesterwachse, Sasolwachse und hydrierte Jojobawachse, einsetzbar.

Weiterhin geeignet sind die Triglyceride gesättigter und gegebenenfalls hydroxylierter C16-30-Fettsäuren, wie zum Beispiel gehärtete Triglyceridfette (hydriertes Palmöl, hydriertes Kokosöl, hydriertes Rizinusöl), Glyceryltribehenat oder Glyceryltri-12-hydroxystearat.

Die Wachskomponenten können auch aus der Gruppe der Ester aus gesättigten, unverzweigten Alkancarbonsäuren einer Kettenlänge von 26 bis 44 C-Atomen und gesättigten, unverzweigten Alkoholen einer Kettenlänge von 26 bis 44 C-Atomen ausgewählt werden, sofern die Wachskomponente oder die Gesamtheit der Wachskomponenten bei Raumtemperatur fest sind. Auch Silikonwachse, zum Beispiel Stearyltrimethylsilan/Stearylalkohol sind gegebenenfalls vorteilhaft.

Natürliche, chemisch modifizierte und synthetische Wachse können alleine oder in Kombination eingesetzt werden. Es können somit auch mehrere Wachse eingesetzt werden. Weiterhin ist auch eine Reihe von Wachsmischungen, ggf. in Abmischung mit weiteren Zusätzen, im Handel erhältlich. Die unter den Bezeichnungen "Spezialwachs 7686 OE" (eine Mischung aus Cetylpalmitat, Bienenwachs, mikrokristallinem Wachs und Polyethylen mit einem Schmelzbereich von 73-75 °C; Hersteller: Kahl & Co), Polywax^{®} GP 200 (eine Mischung von Stearylalkohol und Polyethylenglykolstearat mit einem Schmelzpunkt von 47-51 °C; Hersteller: Croda) und "Weichceresin^{®} FL 400" (ein Vaseline/Vaselinöl/Wachs-Gemisch mit einem Schmelzpunkt von 50-54 °C; Hersteller: Parafluid Mineralölgesellschaft) sind Beispiele für einsetzbare Mischungen.

Bevorzugt ist das Wachs ausgewählt aus Carnaubawachs (INCI: Copernicia Cerifera Cera) Bienenwachs (INCI: Beeswax), Petrolatum (INCI), mikrokristallinem Wachs und insbesondere Gemischen daraus.

Bevorzugte Mischungen umfassen die Kombination von Carnaubawachs (INCI: Copernicia Cerifera Cera), Petrolatum und mikrokristallinem Wachs oder die Kombination von Bienenwachs (INCI: Beeswax) und Petrolatum.

Das Wachs oder die Wachskomponenten sollten bei 25 °C fest sein und sollen im Bereich von > 37 °C schmelzen

Das Mittel zur Behandlung eines keratinischen Materials enthält die festigende Verbindung vorzugsweise in einer Gesamtmenge von 0,5 bis 50 Gew.-%, bevorzugt 1 bis 40 Gew.-%, weiter bevorzugt 1,5 bis 30 Gew.-%, noch mehr bevorzugt 2 bis 25 Gew.-%, bezogen auf das Gesamtgewicht der kosmetischen Zusammensetzung.

Weitere geeignete Inhaltsstoffe umfassen nichtionische Polymere, anionische Polymere, (weitere) kationische Polymere, Wachse, Proteinhydrolysate, Aminosäuren, Oligopetide, Vitamine, Provitamine, Vitaminvorstufen, Betaine, Biochinone, Purin(derivate), Pflegestoffe, Pflanzenextrakte, Silikone, Esteröle, UV-Lichtschutzfilter, Strukturierungsmittel, Verdickungsmittel, Elektrolyte, pH-Stellmittel, Quellmittel, Farbstoffe, Antischuppenwirkstoffe, Komplexbildner, Trübungsmittel, Perlglanzmittel, Pigmente, Stabilisierungsmittel, Treibmittel, Antioxidantien, Parfümöle und/oder Konservierungsmittel.

In den bevorzugten Ausführungsformen 1 bis 20 werden die organischen Siliciumverbindungen der folgenden Tabelle mit einem Tensid der folgenden Tabelle in einem erfindungsgemäßen kosmetischen Mittel miteinander kombiniert. Die Alkylgruppen der aufgeführten Tenside umfassen 8 bis 24, bevorzugt 12 bis 22, bevorzugter 16 bis 18 C-Atome. In den Fällen, in denen ein Tensid zwei Alkylgruppen umfasst, weist mindestens eine Alkylgruppe 8 bis 24, bevorzugt 12 bis 22, bevorzugter 16 bis 18 C-Atome auf, während die weitere Alkylgruppe 1 bis 10, bevorzugt 2 bis 4 C-Atome aufweisen kann. Der Ausdruck "Ether" steht für alkoxylierte, vorzugsweise ethoxylierte, Tenside. Der Ausdruck "Alkoxylat" umfasst insbesondere Ethoxylate und/oder Propoxylate und/oder Butoxylate.

| | Silanverbindung | Tensid |
|---|---|---|
| 1 | (3-Aminopropyl)trimethoxysilan + Methyltrimethoxysilan | Alkylcarbonsäure(salz) |
| 2 | (3-Aminopropyl)triethoxysilan + Methyltriethoxysilan | Alkylcarbonsäure(salz) |
| 3 | (3-Aminopropyl)trimethoxysilan + Ethyltrimethoxysilan | Alkylcarbonsäure(salz) |
| 4 | (3-Aminopropyl)triethoxysilan + Ethyltriethoxysilan | Alkylcarbonsäure(salz) |
| 5 | (3-Aminopropyl)trimethoxysilan + Methyltrimethoxysilan | Alkyl(ether)sulfat |
| 6 | (3-Aminopropyl)triethoxysilan + Methyltriethoxysilan | Alkyl(ether)sulfat |
| 7 | (3-Aminopropyl)trimethoxysilan + Ethyltrimethoxysilan | Alkyl(ether)sulfat |
| 8 | (3-Aminopropyl)triethoxysilan + Ethyltriethoxysilan | Alkyl(ether)sulfat |
| 9 | (3-Aminopropyl)trimethoxysilan + Methyltrimethoxysilan | Alkylbetain |
| 10 | (3-Aminopropyl)triethoxysilan + Methyltriethoxysilan | Alkylbetain |
| 11 | (3-Aminopropyl)trimethoxysilan + Ethyltrimethoxysilan | Alkylbetain |
| 12 | (3-Aminopropyl)triethoxysilan + Ethyltriethoxysilan | Alkylbetain |
| 13 | (3-Aminopropyl)trimethoxysilan + Methyltrimethoxysilan | Alkylglucosid |
| 14 | (3-Aminopropyl)triethoxysilan + Methyltriethoxysilan | Alkylglucosid |
| 15 | (3-Aminopropyl)trimethoxysilan + Ethyltrimethoxysilan | Alkylglucosid |
| 16 | (3-Aminopropyl)triethoxysilan + Ethyltriethoxysilan | Alkylglucosid |
| 17 | (3-Aminopropyl)trimethoxysilan + Methyltrimethoxysilan | Alkylalkoholalkoxylat |
| 18 | (3-Aminopropyl)triethoxysilan + Methyltriethoxysilan | Alkylalkoholalkoxylat |
| 19 | (3-Aminopropyl)trimethoxysilan + Ethyltrimethoxysilan | Alkylalkoholalkoxylat |
| 20 | (3-Aminopropyl)triethoxysilan + Ethyltriethoxysilan | Alkylalkoholalkoxylat |

In diesen bevorzugten Ausführungsformen können ferner bevorzugt noch ein weiteres Tensid in den kosmetischen Mitteln enthalten sein. Besonders bevorzugt wird im Falle eines anionischen Tensids als weiteres Tensid ein nichtionisches Tensid oder ein amphoteres Tensid eingesetzt.

Die Wirkstoffkombination aus mindestens einer organischen Siliciumverbindung und den anionischen, nichtionischen und/oder amphoteren Tensiden kann bereits im Mittel zur Behandlung eines keratinischen Materials enthalten sein. In dieser Ausführungsform wird das Mittel zur Behandlung eines keratinischen Materials bereits in anwendungsbereiter Form vertrieben. Um eine möglichst lagerstabile Formulierung bereitstellen zu können, wird das Mittel selbst bevorzugt wasserarm oder wasserfrei konfektioniert.

Alternativ werden die organischen Siliciumverbindungen maximal 12 Stunden, bevorzugt maximal 6 Stunden, mehr bevorzugt maximal 1 Stunde, noch mehr bevorzugt maximal 30 Minuten und ganz besonders bevorzugt maximal 20 Minuten vor Anwendung des Mittels zur Behandlung eines keratinischen Materials einer Basis umfassend alle Inhaltsstoffe des Mittels zur Behandlung eines keratinischen Materials mit Ausnahme der organischen Siliciumverbindungen zugefügt.

Beispielsweise kann der Anwender ein Mittel (α), welches die organischen Siliciumverbindungen enthält, zunächst mit einem Mittel (β), welches die restlichen Inhaltsstoffe des Mittels zur Behandlung eines keratinischen Materials umfasst, verrühren oder verschütteln. Diese Mischung aus (α) und (β) kann der Anwender nun - entweder direkt nach ihrer Herstellung oder nach einer kurzen Reaktionszeit von 1 Minute bis 20 Minuten - auf die keratinischen Materialien applizieren. Das Mittel (β) kann Wasser enthalten, insbesondere Wasser in einer Menge > 30 Gew.-%, bezogen auf das Gesamtgewicht des Mittels zur Behandlung keratinischer Materialien.

Ein weiterer Gegenstand der vorliegenden Anmeldung ist die Verwendung eines erfindungsgemäßen kosmetischen Mittels zur Behandlung eines keratinischen Materials
zur Pflege von keratinischem Material,
zur Reduzierung und/oder Verhinderung des Verblassens von oxidativ gefärbtem keratinischen Materials,
zur Hydrophobisierung der Oberfläche von keratinischem Material, und/oder
zur Verbesserung der Kämmbarkeit von keratinischem Material.

Bezüglich weiterer bevorzugter Ausführungsformen der Verwendung gilt mutatis mutandis das zu den kosmetischen Mitteln Gesagte.

## Patentansprüche

1. Kosmetisches Mittel zur Behandlung eines keratinischen Materials, umfassend
a) mindestens eine organische Siliciumverbindung und
b) ein anionisches, nichtionisches und/oder amphoteres Tensid,
wobei das Mittel zur Behandlung eines keratinischen Materials- bezogen auf das Gesamtgewicht des Mittels zur Behandlung eines keratinischen Materials - enthält: - 0,5 bis 3 Gew.-% mindestens einer ersten organischen Siliciumverbindung, die ausgewählt ist aus der Gruppe aus (3-Aminopropyl)trimethoxysilan, (3-Aminopropyl)triethoxysilan, (2-Aminoethyl)trimethoxysilan, (2-Aminoethyl)triethoxysilan, (3-Dimethylaminopropyl)-trimethoxysilan, (3-Dimethylaminopropyl)triethoxysilan (2-Dimethylaminoethyl)trimethoxysilan und (2-Dimethylaminoethyl)triethoxysilan, und - 3,2 bis 7 Gew.-% mindestens einer zweiten organischen Siliciumverbindung, die ausgewählt ist aus der Gruppe aus Methyltrimethoxysilan, Methyltriethoxysilan, Ethyltrimethoxysilan, Ethyltriethoxysilan, Propyltrimethoxysilan, Propyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan, Octadecyltrimethoxysilan und Octadecyltriethoxysilan.

2. Kosmetisches Mittel zur Behandlung eines keratinischen Materials gemäß Anspruch 1,
**dadurch gekennzeichnet,**
**dass** die mindestens eine organische Siliciumverbindung ferner eine Verbindung der Formel (II) enthält,
wobei in der organischen Siliciumverbindung der Formel (II)
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c} (II),
- R₅, R_{5'}, R_{5"}, R₆, R_{6'} und R_{6"} unabhängig voneinander für eine C₁-C₆-Alkylgruppe stehen,
- A, A', A", A‴ und Aʺʺ unabhängig voneinander für eine lineare oder verzweigte, zweibindige C₁-C₂₀-Alkylengruppe stehen,
- R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-Alkylgruppe oder eine Gruppierung der Formel (III) stehen
- (Aʺʺ)-Si(R₆")_{d"}(OR₅")_{c"} (III),
- c für eine ganze Zahl von 1 bis 3 steht,
- d für die ganze Zahl 3 - c steht,
- c' für eine ganze Zahl von 1 bis 3 steht,
- d' für die ganze Zahl 3 - c' steht,
- c" für eine ganze Zahl von 1 bis 3 steht,
- d" für die ganze Zahl 3 - c" steht,
- e für 0 oder 1 steht,
- f für 0 oder 1 steht,
- g für 0 oder 1 steht,
- h für 0 oder 1 steht,
mit der Maßgabe, dass mindestens einer der Reste aus e, f, g und h von 0 verschieden ist.

3. Kosmetisches Mittel zur Behandlung eines keratinischen Materials gemäß einem Anspruch 2,
**dadurch gekennzeichnet, dass** das Mittel zur Behandlung eines keratinischen Materials mindestens eine organische Siliciumverbindung der Formel (II) enthält, die ausgewählt ist aus der Gruppe aus
- 3-(Trimethoxysilyl)-N-[3-(trimethoxysilyl)propyl]-1-propanamin
- 3-(Triethoxysilyl)-N-[3-(triethoxysilyl)propyl]-1-propanamin
- N-Methyl-3-(trimethoxysilyl)-N-[3-(trimethoxysilyl)propyl]-1-propanamin
- N-Methyl-3-(triethoxysilyl)-N-[3-(triethoxysilyl)propyl]-1-propanamin
- 2-[Bis[3-(trimethoxysilyl)propyl]amino]-ethanol
- 2-[Bis[3-(triethoxysilyl)propyl]amino]-ethanol
- 3-(Trimethoxysilyl)-N,N-bis[3-(trimethoxysilyl)propyl]-1-Propanamin
- 3-(Triethoxysilyl)-N,N-bis[3-(triethoxysilyl)propyl]-1-Propanamin
- N1,N1-Bis[3-(trimethoxysilyl)propyl]-1,2-Ethanediamin,
- N1,N1-Bis[3-(triethoxysilyl)propyl]-1,2-Ethanediamin,
- N,N-Bis[3-(trimethoxysilyl)propyl]-2-Propen-1-amin und
- N,N-Bis[3-(triethoxysilyl)propyl]-2-Propen-1-amin.

4. Kosmetisches Mittel zur Behandlung eines keratinischen Materials gemäß einem der Ansprüche 1 bis 3, **dadurch gekennzeichnet, dass** die organische Siliciumverbindung in einer Menge von 0,01 bis 10 Gew.-%, bevorzugt von 0,02 bis 8 Gew.-%, bevorzugter von 0,05 bis 6 Gew.-%, am meisten bevorzugt von 0,1 bis 4 Gew.-%, bezogen auf das Gesamtgewicht des kosmetischen Mittels, in dem kosmetischen Mittel enthalten ist.

5. Kosmetisches Mittel zur Behandlung eines keratinischen Materials gemäß einem der Ansprüche 1 bis 4, **dadurch gekennzeichnet, dass** das anionische Tensid ausgewählt ist aus der Gruppe bestehend aus
- geradkettigen oder verzweigten, gesättigten oder ein- oder mehrfach ungesättigten Alkylsulfonaten mit 8 bis 24, bevorzugt 12 bis 22, bevorzugter 16 bis 18 C-Atomen,
- linearen Alpha-Olefinsulfonaten mit 8 bis 24, bevorzugt 12 bis 22, bevorzugter 16 bis 18 C-Atomen,
- Alkylsulfaten und Alkylpolyglykolethersulfaten der Formel R₉-O-(CH₂-CH₂O)ₙ-SO₃X, in der R₉ bevorzugt für einen geradkettigen oder verzweigten, gesättigten oder ein- oder mehrfach ungesättigten Alkyl- oder Alkenlyrest mit 8 bis 24, bevorzugt 12 bis 22, bevorzugter 16 bis 18 Kohlenstoffatomen, n für 0 oder 1 bis 12 , bevorzugter 2 bis 4, und X für ein Alkali- oder Erdalkalimetallion oder für protoniertes Triethanolamin oder das Ammonium-Ion steht,
- geradkettigen oder verzweigten, gesättigten oder ein- oder mehrfach ungesättigten Alkylcarbonsäuren mit 8 bis 24, bevorzugt 12 bis 22, bevorzugter 16 bis 18 C-Atomen,
- geradkettigen oder verzweigten, gesättigten oder ein- oder mehrfach ungesättigten Alkylphosphaten mit 8 bis 24, bevorzugt 12 bis 22, bevorzugter 16 bis 18 C-Atomen,
- Alkylisethionat, dessen Alkylgruppe ausgewählt ist aus einer verzweigten oder unverzweigten C₆ bis C₂₂, bevorzugt C₁₀ bis C₁₈, bevorzugter C₁₂ bis C₁₆ Alkylgruppe, insbesondere Natriumcocoylisethionat,
- Alkylglycosidcarbonsäuren, dessen Alkylgruppe ausgewählt ist aus einer verzweigten oder unverzweigten C₆ bis C₂₂, bevorzugt C₁₀ bis C₁₈, bevorzugter C₁₂ bis C₁₆ Alkylgruppe,
- Alkylsulfosuccinaten, dessen zwei Alkylgruppen ausgewählt sind aus gleichen oder verschiedenen, verzweigte oder unverzweigten C₂ bis C₁₂, bevorzugt C₄ bis C₁₀, bevorzugter C₆ bis C₈ Alkylgruppen,
- Alkyltauraten, dessen Alkylgruppe ausgewählt ist aus einer verzweigten oder unverzweigten C₆ bis C₂₂, bevorzugt C₁₀ bis C₁₈, bevorzugter C₁₂ bis C₁₆ Alkylgruppe,
- Alkylsarcosinaten, dessen Alkylgruppe ausgewählt ist aus einer verzweigten oder unverzweigten C₆ bis C₂₂, bevorzugt C₁₀ bis C₁₈, bevorzugter C₁₂ bis C₁₆ Alkylgruppe, und
- Sulfonate ungesättigter Fettsäuren mit 8 bis 24, bevorzugt 12 bis 22, bevorzugter 16 bis 18 C-Atomen und 1 bis 6 Doppelbindungen,
wobei das Gegenion des anionischen Tensids ein Alkali- oder Erdalkalimetallion oder ein protoniertes Triethanolamin oder das Ammonium-Ion ist, insbesondere ist das anionische Tensid Natriumlaurylethersulfat (INCI: Sodium Laureth Sulfate).

6. Kosmetisches Mittel zur Behandlung eines keratinischen Materials gemäß einem der Ansprüche 1 bis 5, **dadurch gekennzeichnet, dass** das amphotere Tensid ausgewählt ist aus der Gruppe bestehend aus
- Alkylbetain, umfassend mindestens eine gesättigte oder ungesättigte, verzweigte oder unverzweigte C₆ bis C₂₂, bevorzugt C₁₀ bis C₁₈, bevorzugter C₁₂ bis C₁₆ Alkylgruppe,
- Alkylamphodiacetat oder Alkylamphodiacetat, umfassend eine gesättigte oder ungesättigte, verzweigte oder unverzweigte C₆ bis C₂₂, bevorzugt C₁₀ bis C₁₈, bevorzugter C₁₂ bis C₁₆ Alkylgruppe, mit einem Alkali- oder Erdalkalimetallgegenion, und
- Alkylamidopropylbetain, umfassend mindestens eine gesättigte oder ungesättigte, verzweigte oder unverzweigte C₆ bis C₂₂, bevorzugt C₁₀ bis C₁₈, bevorzugter C₁₂ bis C₁₆ Alkylgruppe,
wobei das amphotere Tensid insbesondere Cocoamidopropyl-Betain (INCI) oder Disodium Cocoamphodiacetat (INCI) ist.

7. Kosmetisches Mittel zur Behandlung eines keratinischen Materials gemäß einem der Ansprüche 1 bis 6, **dadurch gekennzeichnet, dass** das nichtionische Tensid ausgewählt ist aus der Gruppe bestehend aus
- Alkylglucamid, umfassend eine gesättigte oder ungesättigte, verzweigte oder unverzweigte C₆ bis C₂₂, bevorzugt C₁₀ bis C₁₈, bevorzugter C₁₂ bis C₁₆ Alkylgruppe,
- Alkylglucosid, umfassend eine gesättigte oder ungesättigte, verzweigte oder unverzweigte C₆ bis C₂₂, bevorzugt C₁₀ bis C₁₈, bevorzugter C₁₂ bis C₁₆ Alkylgruppe,
- Alkylfructosid, umfassend eine gesättigte oder ungesättigte, verzweigte oder unverzweigte C₆ bis C₂₂, bevorzugt C₁₀ bis C₁₈, bevorzugter C₁₂ bis C₁₆ Alkylgruppe, und
- Alkylalkoholalkoxylat der Formel R₁₀(OR₁₁)ₘOH, in der R₁₀ eine lineare oder verzweigte C₆ bis C₂₂, bevorzugt C₁₀ bis C₁₈, bevorzugter C₁₂ bis C₁₆ Alkylgruppe, R₁₁ eine C₂ bis C₄, bevorzugt eine C₂ Alkylgruppe, und m 1 bis 10, bevorzugt 2 bis 6, bevorzugter 2 bis 6, darstellen, und
- Alkylester der Formel R₁₂COOR₁₃, in der R₁₂ eine lineare oder verzweigte C₆ bis C₂₂, bevorzugt C₁₀ bis C₁₈, bevorzugter C₁₂ bis C₁₆ Alkylgruppe, R₁₃ eine C₁ bis C₄, bevorzugt eine C₂ Alkylgruppe, darstellen.

8. Kosmetisches Mittel zur Behandlung eines keratinischen Materials gemäß einem der Ansprüche 1 bis 7, **dadurch gekennzeichnet, dass** das kosmetische Mittel zwei strukturell voneinander verschiedene Tenside enthält, bevorzugt **dadurch gekennzeichnet, dass** das kosmetische Mittel zwei strukturell voneinander verschiedene anionische Tenside enthält, oder dass das kosmetische Mittel ein anionisches Tensid und ein nichtionisches Tensid enthält, oder dass das kosmetische Mittel ein anionisches Tensid und ein amphoteres Tensid enthält.

9. Kosmetisches Mittel zur Behandlung eines keratinischen Materials gemäß einem der Ansprüche 1 bis 8, **dadurch gekennzeichnet, dass** das anionische, nichtionische und/oder amphotere Tensid eine Gesamtmenge an Tensid bilden, wobei die Gesamtmenge an Tensid von 1 bis 30 Gew.-%, bevorzugt von 2 bis 25 Gew.-%, bevorzugter von 3 bis 20 Gew.-%, beträgt, bezogen auf das Gesamtgewicht des kosmetischen Mittels.

10. Verwendung eines kosmetischen Mittels zur Behandlung eines keratinischen Materials gemäß einem der Ansprüche 1 bis 9
zur Pflege von keratinischem Material,
zur Reduzierung und/oder Verhinderung des Verblassens von oxidativ gefärbtem keratinischen Materials,
zur Hydrophobisierung der Oberfläche von keratinischem Material und/oder zur Verbesserung der Kämmbarkeit von keratinischem Material.

## Claims

1. A cosmetic composition for treating keratinous material, comprising
a) at least one organic silicon compound and
b) an anionic, nonionic, and/or amphoteric surfactant,
wherein the cosmetic composition for treating keratinous material-based on the total weight of the cosmetic composition for treating keratinous material-contains: - 0.5 to 3% by weight of at least one first organic silicon compound selected from the group consisting of (3-aminopropyl)trimethoxysilane, (3-aminopropyl)triethoxysilane, (2-aminoethyl)trimethoxysilane, (2-aminoethyl)triethoxysilane, (3-dimethylaminopropyl)trimethoxysilane, (3-dimethylaminopropyl)triethoxysilane, (2-dimethylaminoethyl)trimethoxysilane, and (2-dimethylaminoethyl)triethoxysilane, and - 3.2 to 7 wt% of at least one second organic silicon compound selected from the group consisting of methyltrimethoxysilane, methyltriethoxysilane, ethyltrimethoxysilane, ethyltriethoxysilane, propyltrimethoxysilane, propyltriethoxysilane, hexyltrimethoxysilane, hexyltriethoxysilane, octyltrimethoxysilane, octyltriethoxysilane, dodecyltrimethoxysilane, dodecyltriethoxysilane, octadecyltrimethoxysilane, and octadecyltriethoxysilane.

2. A cosmetic composition for treating keratinous material according to claim 1,
**characterized in**
**that** the at least one organic silicon compound further comprises a compound of formula (II),
wherein, in the organic silicon compound of formula (II),
(R₅O)_{c}(R₆)_{d} Si-(A)ₑ -[NR₇-(A')]_{f}-[O-(A")]_{g} -[NR₈-(A‴)]ₕ -Si(R₆')_{d'}(OR₅')_{c'} (II),
- R₅, R_{5'}, R_{5"}, R₆, R_{6'} and R_{6"} independently represent a C₁-C₆-alkyl group,
- A, A', A", A‴, and Aʺʺ independently represent a linear or branched, divalent C₁ -C₂₀-alkylene group,
- R₇ and R₈ independently represent a hydrogen atom, a C₁-C₆-alkyl group, a hydroxy-C₁ -C₆-alkyl group, a C₂-C₆-alkenyl group, an amino-C₁-C₆-alkyl group, or a group of formula (III)
- (Aʺʺ)-Si(R₆")_{d"} (OR₅")_{c"} (III),
- where c is an integer from 1 to 3,
- d represents the integer 3 - c,
- c' stands for an integer from 1 to 3,
- d' stands for the integer 3 - c',
- c" represents an integer from 1 to 3,
- d" stands for the integer 3 - c",
- e stands for 0 or 1,
- f stands for 0 or 1,
- g stands for 0 or 1,
- h is 0 or 1,
provided that at least one of the radicals e, f, g, and h is not 0.

3. A cosmetic composition for treating a keratinous material according to claim 2, **characterized in that** the composition for treating a keratinous material contains at least one organic silicon compound of formula (II) selected from the group consisting of
- 3-(trimethoxysilyl)-N-[3-(trimethoxysilyl)propyl]-1-propanamine
- 3-(triethoxysilyl)-N-[3-(triethoxysilyl)propyl]-1-propanamine
- N-methyl-3-(trimethoxysilyl)-N-[3-(trimethoxysilyl)propyl]-1-propanamine
- N-Methyl-3-(triethoxysilyl)-N-[3-(triethoxysilyl)propyl]-1-propanamine
- 2-[Bis[3-(trimethoxysilyl)propyl]amino]ethanol
- 2-[Bis[3-(triethoxysilyl)propyl]amino]ethanol
- 3-(Trimethoxysilyl)-N,N-bis[3-(trimethoxysilyl)propyl]-1-propanamine
- 3-(Triethoxysilyl)-N,N-bis[3-(triethoxysilyl)propyl]-1-propanamine
- N1,N1-Bis[3-(trimethoxysilyl)propyl]-1,2-ethanediamine,
- N1,N1-Bis[3-(triethoxysilyl)propyl]-1,2-ethanediamine,
- N,N-Bis[3-(trimethoxysilyl)propyl]-2-propen-1-amine, and
- N,N-bis[3-(triethoxysilyl)propyl]-2-propen-1-amine.

4. A cosmetic composition for treating a keratinous material according to any one of claims 1 to 3, **characterized in that** the organic silicon compound is contained in the cosmetic composition in an amount of 0.01 to 10 wt.%, preferably 0.02 to 8 wt.%, more preferably 0.05 to 6 wt.%, most preferably from 0.1 to 4% by weight, based on the total weight of the cosmetic composition, in the cosmetic composition.

5. Cosmetic composition for treating keratinous material according to any one of claims 1 to 4, **characterized in that** the anionic surfactant is selected from the group consisting of
- straight-chain or branched, saturated or mono- or polyunsaturated alkyl sulfonates having 8 to 24, preferably 12 to 22, more preferably 16 to 18 carbon atoms,
- linear alpha-olefin sulfonates having 8 to 24, preferably 12 to 22, more preferably 16 to 18 carbon atoms,
- alkyl sulfates and alkyl polyglycol ether sulfates of the formula R₉-O-(CH₂-CH₂-O)ₙ-SO₃X, wherein R₉ preferably represents a straight-chain or branched, saturated or mono- or polyunsaturated alkyl or alkenyl radical having 8 to 24, preferably 12 to 22, more preferably 16 to 18 carbon atoms, n represents 0 or 1 to 12, preferably 2 to 4, and X stands for an alkali or alkaline earth metal ion or for protonated triethanolamine or the ammonium ion,
- straight-chain or branched, saturated or mono- or polyunsaturated alkylcarboxylic acids having 8 to 24, preferably 12 to 22, more preferably 16 to 18 carbon atoms,
- straight-chain or branched, saturated or mono- or polyunsaturated alkyl phosphates having 8 to 24, preferably 12 to 22, more preferably 16 to 18 carbon atoms,
- an alkyl isethionate in which the alkyl group is selected from a branched or unbranched C₆ to C₂₂ alkyl group, preferably a C₁₀ to C₁₈ alkyl group, more preferably a C₁₂ to C₁₆ alkyl group, in particular sodium cocoyl isethionate,
- Alkyl glycoside carboxylic acids, wherein the alkyl group is selected from a branched or unbranched C₆ to C₂₂, preferably C₁₀to C₁₈, more preferably C₁₂ to C₁₆ alkyl group,
- alkyl sulfosuccinates, whose two alkyl groups are selected from identical or different, branched or unbranched C₂t o C₁₂, preferably C₄ to C₁₀, more preferably C₆ to C₈ alkyl groups,
- alkyltaurates, whose alkyl group is selected from a branched or unbranched C₆ to C₂₂, preferably C₁₀ to C₁₈, more preferably C₁₂ to C₁₆ alkyl group,
- alkyl sarcosinates, wherein the alkyl group is selected from a branched or unbranched C₆ to C₂₂, preferably C₁₀ to C₁₈, more preferably C₁₂ to C₁₆ alkyl group, and
- sulfonates of unsaturated fatty acids having 8 to 24, preferably 12 to 22, more preferably 16 to 18 carbon atoms and 1 to 6 double bonds,
wherein the counterion of the anionic surfactant is an alkali or alkaline earth metal ion or a protonated triethanolamine or the ammonium ion; in particular, the anionic surfactant is sodium lauryl ether sulfate (INCI: Sodium Laureth Sulfate).

6. A cosmetic composition for treating keratinous material according to any one of claims 1 to 5, **characterized in that** the amphoteric surfactant is selected from the group consisting of
- alkyl betaine, comprising at least one saturated or unsaturated, branched or unbranched C₆ to C₂₂, preferably C₁₀ to C₁₈ , more preferably C₁₂ to C₁₆ alkyl group,
- alkylamphodiacetate or alkylamphodiacetate comprising a saturated or unsaturated, branched or unbranched C₆ to C₂₂, preferably C₁₀ to C₁₈, more preferably C₁₂ to C₁₆ alkyl group, with an alkali or alkaline earth metal counterion, and
- An alkylamidopropyl betaine comprising at least one saturated or unsaturated, branched or unbranched C₆ to C₂₂ alkyl group, preferably C₁₀ to C₁₈, more preferably C₁₂ to C₁₆,
wherein the amphoteric surfactant is, in particular, cocoamidopropyl betaine (INCI) or disodium cocoamphodiacetate (INCI).

7. A cosmetic composition for treating keratinous material according to any one of claims 1 to 6, **characterized in that** the nonionic surfactant is selected from the group consisting of
- alkylglucamide, comprising a saturated or unsaturated, branched or unbranched C₆ to C₂₂, preferably C₁₀ to C₁₈, more preferably C₁₂ to C₁₆ alkyl group,
- alkyl glucoside comprising a saturated or unsaturated, branched or unbranched C₆ to C₂₂, preferably C₁₀ to C₁₈, more preferably C₁₂ to C₁₆ alkyl group,
- alkyl fructoside comprising a saturated or unsaturated, branched or unbranched C₆ to C₂₂, preferably C₁₀ to C₁₈, more preferably C₁₂ to C₁₆ alkyl group, and
- an alkyl alcohol alkoxylate of the formula R₁₀ (OR₁₁)ₘ OH, wherein R₁₀ is a linear or branched C₆ to C₂₂ , preferably C₁₀ to C₁₈ , more preferably C₁₂ to C₁₆ alkyl group, R₁₁ represents a C₂ to C₄ , preferably a C₂ alkyl group, and m represents 1 to 10, preferably 2 to 6, more preferably 2 to 6, and
- alkyl esters of the formula R₁₂COOR₁₃, in which R₍₁₂₎ represents a linear or branched C₆ to C₂₂, preferably C₁₀ to C₁₈, more preferably C₁₂ to C₁₆ alkyl group, R₁₃ represents a C₁ to C₄ , preferably a C₂ alkyl group.

8. A cosmetic composition for treating keratinous material according to any one of claims 1 through 7, **characterized in that** the cosmetic product contains two structurally different surfactants, preferably **characterized in that** the cosmetic product contains two structurally different anionic surfactants, or that the cosmetic product contains an anionic surfactant and a nonionic surfactant, or that the cosmetic product contains an anionic surfactant and an amphoteric surfactant.

9. A cosmetic composition for treating keratinous material according to any one of claims 1 to 8, **characterized in that** the anionic, nonionic, and/or amphoteric surfactant form a total amount of surfactant, wherein the total amount of surfactant is from 1 to 30 wt.%, preferably from 2 to 25 wt.%, more preferably from 3 to 20 wt.%, based on the total weight of the cosmetic composition.

10. Use of a cosmetic composition for the treatment of keratinous material according to any one of claims 1 to 9
for the care of keratinous material,
for reducing and/or preventing the fading of oxidatively colored keratinous material,
for hydrophobizing the surface of keratinous material and/or
for improving the combability of keratinous material.

## Revendications

1. Produit cosmétique destiné au traitement d'une matière kératinique, comprenant
a) au moins un composé organique du silicium et
b) un tensioactif anionique, non ionique et/ou amphotère,
le produit de traitement d'une matière kératinique contenant, par rapport au poids total du produit de traitement d'une matière kératinique :
- 0,5 à 3 % en poids d'au moins un premier composé organique du silicium choisi dans le groupe constitué par le (3-aminopropyl)triméthoxysilane, le (3-aminopropyl)triéthoxysilane, le (2-aminoéthyl)triméthoxysilane, le (2-aminoéthyl)triéthoxysilane, le (3-diméthylaminopropyl)triméthoxysilane, le (3-diméthylaminopropyl)triéthoxysilane, le (2-diméthylaminoéthyl)triméthoxysilane et le (2-diméthylaminoéthyl)triéthoxysilane, et
- 3,2 à 7 % en poids d'au moins un deuxième composé organique du silicium choisi dans le groupe constitué par le méthyltriméthoxysilane, le méthyltriéthoxysilane, éthyltriméthoxysilane, éthyltriéthoxysilane, propyltriméthoxysilane, propyltriéthoxysilane, hexyltriméthoxysilane, hexyltriéthoxysilane, octyltriméthoxysilane, l'octyltriéthoxysilane, le dodécyltriméthoxysilane, le dodécyltriéthoxysilane, l'octadécyltriméthoxysilane et l'octadécyltriéthoxysilane.

2. Produit cosmétique destiné au traitement d'une matière kératinique selon la revendication 1, caractérisé en ce
en ce que ledit au moins un composé organique du silicium contient en outre un composé de formule (II),
dans lequel, dans le composé organique du silicium de formule (II)
(R₅O)_{c}(R₆)_{d}Si-(A)ₑ-[NR₇-(A')]_{f} -[O-(A")]_{g} -[NR₈-(A‴)]ₕ-Si(R₆')_{d'}(OR₅')_{c'} (II),
- R₅ , R_{5'} , R_{5"} , R₆ , R_{6'} et R_{6"} représentent, indépendamment les uns des autres, un groupe alkyle en C₁-C₆,
- A, A', A", A"' et Aʺʺ représentent, indépendamment les uns des autres, un groupe alkylène en C₁-C₂₀ linéaire ou ramifié à deux liaisons,
- R₇ et R₈ représentent, indépendamment l'un de l'autre, un atome d'hydrogène, un groupe alkyle en C₁ -C₆ , un groupe hydroxy-C₁ -C₆-alkyle, un groupe alcényle en C₂-C₆, un groupe amino-C₁-C₆-alkyle ou un groupement de formule (III)
- (Aʺʺ)-Si(R₆ ")_{d'} (OR₅")_{c"} (III),
- c représente un nombre entier compris entre 1 et 3,
- d représente le nombre entier 3 - c,
- c' représente un nombre entier compris entre 1 et 3,
- d' représente l'entier 3 - c',
- c" représente un nombre entier compris entre 1 et 3,
- d" représente le nombre entier 3 - c",
- e représente 0 ou 1,
- f représente 0 ou 1,
- g représente 0 ou 1,
- h représente 0 ou 1,
à condition qu'au moins l'un des radicaux parmi e, f, g et h soit différent de 0.

3. Produit cosmétique destiné au traitement d'une matière kératinique selon la revendication 2, **caractérisé en ce que** le produit destiné au traitement d'une matière kératinique contient au moins un composé organique du silicium de formule (II) choisi dans le groupe constitué par
- 3-(triméthoxysilyl)-N-[3-(triméthoxysilyl)propyl]-1-propanamine
- la 3-(triéthoxysilyl)-N-[3-(triéthoxysilyl)propyl]-1-propanamine
- N-méthyl-3-(triméthoxysilyl)-N-[3-(triméthoxysilyl)propyl]-1-propanamine
- N-méthyl-3-(triéthoxysilyl)-N-[3-(triéthoxysilyl)propyl]-1-propanamine
- 2-[bis[3-(triméthoxysilyl)propyl]amino]-éthanol
- 2-[bis[3-(triéthoxysilyl)propyl]amino]-éthanol
- 3-(triméthoxysilyl)-N,N-bis[3-(triméthoxysilyl)propyl]-1-propanamine
- 3-(triéthoxysilyl)-N,N-bis[3-(triéthoxysilyl)propyl]-1-propanamine
- N1,N1-bis[3-(triméthoxysilyl)propyl]-1,2-éthanediamine,
- N1,N1-bis[3-(triéthoxysilyl)propyl]-1,2-éthanediamine,
- N,N-bis[3-(triméthoxysilyl)propyl]-2-propène-1-amine et
- N,N-bis[3-(triéthoxysilyl)propyl]-2-propène-1-amine.

4. Produit cosmétique destiné au traitement d'une matière kératinique selon l'une des revendications 1 à 3, **caractérisé en ce que** le composé organique du silicium est présent dans le produit cosmétique en une quantité de 0,01 à 10 % en poids, de préférence de 0,02 à 8 % en poids, de manière encore plus préférée de 0,05 à 6 % en poids, de préférence encore de 0,1 à 4 % en poids, par rapport au poids total du produit cosmétique, dans le produit cosmétique.

5. Produit cosmétique destiné au traitement d'une matière kératinique selon l'une des revendications 1 à 4, **caractérisé en ce que** l'agent tensioactif anionique est choisi dans le groupe constitué par
- des alkylsulfonates à chaîne linéaire ou ramifiée, saturés ou mono- ou polyinsaturés, comportant de 8 à 24, de préférence de 12 à 22, et de préférence de 16 à 18 atomes de carbone,
- des alpha-oléfinesulfonates linéaires comportant 8 à 24, de préférence 12 à 22, et de préférence encore 16 à 18 atomes de carbone,
- des alkylsulfates et des alkylpolyglycoléthersulfates de formule R₉-O-(CH₂-CH₂O)ₙ-SO₃X, dans laquelle R₉ représente de préférence un radical alkyle ou alcényle à chaîne droite ou ramifiée, saturé ou mono- ou polyinsaturé, comportant de 8 à 24, de préférence de 12 à 22, et de préférence de 16 à 18 atomes de carbone, n vaut 0 ou 1 à 12, de préférence 2 à 4, et X représente un ion de métal alcalin ou alcalino-terreux, ou la triéthanolamine protonée, ou l'ion ammonium,
- des acides alkylcarboxyliques à chaîne droite ou ramifiée, saturés ou mono- ou polyinsaturés, comportant 8 à 24, de préférence 12 à 22, et de préférence 16 à 18 atomes de carbone,
- des alkylphosphates à chaîne droite ou ramifiée, saturés ou mono- ou polyinsaturés, comportant 8 à 24, de préférence 12 à 22, et de préférence encore 16 à 18 atomes de carbone,
- un iséthionate d'alkyle dont le groupe alkyle est choisi parmi les groupes alkyle ramifiés ou linéaires en C₆ à C₂₂, de préférence en C₁₀ à C₁₈ , et de préférence encore en C₁₂ à C₁₆, en particulier le cocoyliséthionate de sodium,
- des acides alkylglycosidecarboxyliques dont le groupe alkyle est choisi parmi les groupes alkyle ramifiés ou linéaires en C₆ à C₂₂, de préférence en C₁₀ à C₁₈ , et de préférence encore en C₁₂ à C₁₆ ,
- des alkylsulfosuccinates dont les deux groupes alkyle sont choisis parmi des groupes alkyle identiques ou différents, ramifiés ou non ramifiés, en C₂ à C₁₂, de préférence en C₄ à C₁₀, et de préférence en C₆ à C₈,
- des alkyltaurates dont le groupe alkyle est choisi parmi un groupe alkyle en C₆ à C₂₂, de préférence en C₁₀ à C₁₈, de préférence en C₁₂ à C₁₆, ramifié ou non ramifié,
- des alkylsarcosinates dont le groupe alkyle est choisi parmi un groupe alkyle ramifié ou non ramifié en C₆ à C₂₂, de préférence en C₁₀ à C₁₈, et de préférence en C₁₂ à C₁₆, et
- des sulfonates d'acides gras insaturés comportant 8 à 24, de préférence 12 à 22, de préférence 16 à 18 atomes de carbone et 1 à 6 doubles liaisons,
le contre-ion de l'agent tensioactif anionique étant un ion de métal alcalin ou alcalino-terreux, ou une triéthanolamine protonée, ou l'ion ammonium; en particulier, l'agent tensioactif anionique est le lauryléthersulfate de sodium (INCI: Sodium Laureth Sulfate).

6. Produit cosmétique destiné au traitement d'une matière kératinique selon l'une des revendications 1 à 5, **caractérisé en ce que** l'agent tensioactif amphotère est choisi dans le groupe constitué par
- d'alkylbétaïne, comprenant au moins un groupe alkyle en C₆ à C₂₂, de préférence en C₁₀ à C₁₈, de préférence encore en C₁₂ à C₁₆, saturé ou insaturé, ramifié ou non ramifié,
- d'amphodiacétate d'alkyle ou d'amphodiacétate d'alkyle comprenant un groupe alkyle en C₆ à C₂₂, de preference en C₁₀ à C₁₈, de préférence encore en C₁₂ à C₁₆, saturé ou insaturé, ramifié ou non ramifié, avec un contre-ion de métal alcalin ou alcalino-terreux, et
- Alkylamidopropylbétaïne comprenant au moins un groupe alkyle en C₆ à C₂₂, de préférence en C₁₀ à C₁₈, et de préférence encore en C₁₂à C₁₆, saturé ou insaturé, ramifié ou non ramifié,
le tensioactif amphotère étant en particulier la cocoamidopropylbétaïne (INCI) ou le cocoamphodiacétate disodique (INCI).

7. Produit cosmétique destiné au traitement d'une matière kératinique selon l'une des revendications 1 à 6, **caractérisé en ce que** l'agent tensioactif non ionique est choisi dans le groupe constitué par
- d'un alkylglucamide comprenant un groupe alkyle en C₆ à C₂₂, de préférence en C₁₀ à C₁₈, de préférence encore en C₁₂ à C₁₆, saturé ou insaturé, ramifié ou non ramifié,
- d'un alkylglucoside comprenant un groupe alkyle en C₆ à C₂₂, de préférence en C₁₀ à C₁₈, de préférence encore en C₁₂ à C₁₆, saturé ou insaturé, ramifié ou non ramifié,
- alkylfructoside comprenant un groupe alkyle en C₆ à C₂₂, de preference en C₁₀ à C₁₈, de préférence en C₁₂ à C₁₆, saturé ou insaturé, ramifié ou non ramifié, et
- un alcoxylate d'alcool alkylique de formule R₁₀(OR₁₁)ₘOH, dans laquelle R₁₀ représente un groupe alkyle linéaire ou ramifié en C₆ à C₂₂, de préférence en C₁₀ à C₁₈, de préférence en C₁₂ à C₁₆, R₁₁ un groupe alkyle en C₂ à C₄, de preference en C₂, et m 1 à 10, de préférence 2 à 6, de préférence 2 à 6, et
- des esters alkyliques de formule R₁₂COOR₁₃, dans laquelle R₁₂ représente un groupe alkyle linéaire ou ramifié en C₆ à C₂₂, de préférence en C₁₀ à C₁₈, de préférence en C₁₂ à C₁₆ , R₁₃ représentant un groupe alkyle en C₁ à C₄, de préférence en C₂.

8. Produit cosmétique destiné au traitement d'une matière kératinique selon l'une des revendications 1 à 7, **caractérisé en ce que** le produit cosmétique contient deux tensioactifs de structure différente, de préférence **caractérisé en ce que** le produit cosmétique contient deux tensioactifs anioniques de structure différente, ou **en ce que** le produit cosmétique contient un tensioactif anionique et un tensioactif non ionique, ou **en ce que** le produit cosmétique contient un tensioactif anionique et un tensioactif amphotère.

9. Produit cosmétique destiné au traitement d'une matière kératinique selon l'une des revendications 1 à 8, **caractérisé en ce que** l'agent tensioactif anionique, non ionique et/ou amphotère forme une quantité totale d'agent tensioactif, la quantité totale d'agent tensioactif étant comprise entre 1 et 30 % en poids, de préférence entre 2 et 25 % en poids, et de manière encore plus préférée entre 3 et 20 % en poids, par rapport au poids total du produit cosmétique.

10. Utilisation d'un produit cosmétique pour le traitement d'une matière kératinique selon l'une des revendications 1 à 9
pour le soin d'une matière kératinique,
pour réduire et/ou empêcher la décoloration d'une matière kératinique colorée par oxydation,
pour l'hydrophobisation de la surface d'une matière kératinique et/ou
pour améliorer le peignage de la matière kératinique.
